# EUROPEAN PATENT SPECIFICATION

(11) **EP 4 129 363 B1**
(45) Date of publication and mention of the grant of the patent: **08.10.2025**
(21) Application number: 21189326.8
(22) Date of filing: 03.08.2021
(51) Int. Cl.: A61M 5/20, A61M 5/32, A61M 5/31, A61M 5/24

(54) **AUTO INJECTOR WITH DISPOSABLE SYRINGE UNIT**
AUTOMATISCHE INJEKTIONSVORRICHTUNG MIT EINWEGSPRITZENEINHEIT
AUTO-INJECTEUR AVEC UNITÉ DE SERINGUE JETABLE

(43) Date of publication of application: 08.02.2023
(73) Proprietor: Ypsomed AG, 3401 Burgdorf (CH)
(72) Inventor: Steck, Jürg, 3422 Kirchberg (CH); Tschirren, Markus, 3400 Burgdorf (CH); Hirschel, Jürg, 3007 Bern (CH); Jost, Reto, 3506 Grosshöchstetten (CH); Urbanek, Leos, 3012 Bern (CH); Hirniak, Andrew, 8001 Zürich (CH); Merz, Hannes, 4600 Olten (CH); Niederhauser, Aymeric, 2720 Tramelan (CH); Tronza, Mario, 4055 Basel (CH); Weiss, Mario, 3264 Diessbach bei Büren (CH)
(74) Representative: Meier Obertüfer, Jürg

(56) References cited:
- WO-A1-2014/019997
- WO-A1-2019/158372
- WO-A1-2020/070328

## Description

### Technical Field

The invention relates to an auto injector for injection of a medicament having a disposable syringe unit and a reusable drive unit.

### Background Art

The use of syringes to inject liquid medicaments is well known. Syringes rely on pricking the skin of a patient by a hollow needle through which a liquid medicament is delivered to a tissue, muscle or blood vessel of the patient. Typically, syringes comprise a barrel containing a volume of the medicament, a needle coupled to the barrel at a first end thereof as well as a plunger which is axially movably within the barrel to expel the liquid medicament from the barrel through the needle and inject it into the patient.

Also known is the use of auto injectors with syringes. Auto injectors usually comprise a body for housing a syringe as well as a drive mechanism in order to move the plunger of the syringe upon actuation of the auto injector. The drive mechanism typically comprises a source of drive, such as a motor or strong spring for moving a transfer element, for example a rod, which acts on the plunger of the syringe.

For safety and hygiene reasons it is desirable that the needle does not protrude from a housing of the auto injector with the exception of the time when the needle is used for injection of a medicament. Thus, either the auto injector moves the needle out of the housing for the injection and back into the housing after injection or the housing provides a needle guard which may be moved to unsheathe the needle for injection and which may be moved back after the injection.

The majority of auto injectors are configured as single use devices which incorporate both the syringe and the drive mechanism in the same housing. Such devices are usually disposable for hygiene reasons.

Disposable auto injectors comprising an electric actuator or an electronic control require a source of energy which is usually in the form of a battery. However, in this case, the auto injectors should not be disposed in the regular waste, but have to be subjected to special disposal or to recycling, which is an additional burden to the patient. Further, disposing a battery, motor and/or electronics after a single use is a waste of resources and increases the costs of the auto injector.

Hence, auto injectors having a reusable drive unit as well as a syringe unit which may be releasably coupled to the drive unit have been developed. WO 2014/019997 (UCB Pharma S.A.) discloses a disposable cassette which may be used with an auto injector. The cassette comprises a shuttle lock control which is axially movable between a first "cassette unused" position, a second "cassette unlocked" position where locking features of a cap on the cassette are released and a third "cassette used" position which is located between the first and second position. In the first and third position, the locking features of the cap are blocked, so that the cap may not be removed.

WO 2020/070328 (Phillips-Medisize A/S) describes an auto injector which is used with a disposable cassette comprising the syringe. By the proximal movement of pins by a drive the cassette may be locked to the auto injector and a skin sensor may be released to be slidable relative to the cassette. A distal movement of the skin sensor allows to remove a needle sheath such as to unsheathe the needle for injection. The distal movement of the skin sensor compresses a spring which pushes the skin sensor in a proximal position when the needle is retracted from skin. The skin sensor is subsequently locked at a proximal position by means of locking elements, thus permanently sheathing the needle.

WO 2019/158372 (SHL Medical AG) discloses a medicament delivery device with an easily connectable disposable unit to a reusable drive unit. Connection of the disposable unit to the reusable unit is enabled by means of snap-fit elements which may be locked during injection by moving a locking element to block the disengagement of the snap-fit elements.

### Summary of the invention

It is the object of the invention to create an auto injector for injection of a medicament including a reusable drive unit as well as a disposable syringe unit which allows an easy, safe and reliable operation.

The solution of the invention is specified by the features of claim 1. According to the invention the auto injector includes a drive unit as well as a syringe unit which is releasably coupled to the drive unit. The drive unit comprises a longitudinal first housing with a first central axis and with first connection means at a proximal end. An actuator is arranged in said first housing such as to be movable along the first central axis by means of a linear drive of the drive unit. Further, the drive unit comprises a first sleeve which is linearly movable within the first housing along the first central axis, the sleeve being biased towards the proximal end of the first housing by means of a first elastic element. The first sleeve is in contact with a stop surface of the actuator in an initial position thereof, said stop surface limiting the movement of the first sleeve towards the proximal end.

The syringe unit comprises a longitudinal second housing with a second central axis and with second connection means at a distal end thereof, said second connection means being configured to releasably engage the first connection means of the first housing. The second housing further includes third connection means arranged at a proximal end thereof, a syringe arranged within said second housing as well as a cap which is releasably coupled to the proximal end of the second housing by means of fourth connection means, fourth connection means being releasably engaged to the third connection means. A second sleeve is arranged to be linearly movable along the second central axis, said sleeve blocking the release of the engagement of the third connection means with the fourth connection means.

The first sleeve is adapted to block the release of the engagement of the first connection means with the second connection means when the actuator is moved from the initial position to a first position and to abut onto and to push the second sleeve such as to allow a release of the coupling of the third connection means with the fourth connection means when the actuator is moved proximally from the first position to a second position.

The locking of the syringe unit to the drive unit as well as the unlocking of the cap from the syringe unit may be performed by moving only the actuator in the proximal direction, i.e. in a single direction. Further, the use of movable sleeves allows for an even distribution of movement force, thus reducing the risk of failure of a moving part of the auto injector, as well as a more compact configuration of the auto injector.

The auto injector may be used by medical personnel to administer a medicament to a patient or it may be used by a patient for self-administration of a medicament. The medicament may be of any type, such as for example an antibiotic, anticoagulant or the like. More preferably the medicament is a drug for subcutaneous injections an anti-diabetic medicament, such as for example insulin, exenatide, liraglutide or pramlintide.

The drive unit is preferably configured to be re-usable, i.e. to be coupled multiple times with a syringe unit. Hence, the drive unit may be used to inject a medicament multiple times.

The syringe unit is preferably designed as disposable unit for a single time use. Hence, the risk of contamination by re-using an already used syringe unit is avoided. Further, the use of a two part auto-injector allows to provide sterile syringe units without the need to sterilize the drive unit. This opens more options in view of the design of the drive units, as non-sterilizable materials as well as electronic components may be used.

The syringe unit may be coupled to the drive unit prior to the injection and de-coupled from the drive unit after the injection. The syringe unit preferably comprises a volume of the medicament which is sufficient for the administration of a single dose.

In the present application, the term "proximal" means the direction towards the skin of a patient when the auto injector is in use. Accordingly, the term "distal" means the direction away of the skin of a patient when the auto injector is in use. Hence, when an injection is administered to a patient with the auto injector, the auto injector contacts the skin of the patient with its proximal end, specifically with the proximal end of the syringe unit.

The drive unit comprises a longitudinal first housing with a first central axis, said first housing preferably being generally in the shape of a cuboid, preferably with rounded edges. I.e. the housing has a rectangular cross section with rounded corners in this case. Alternatively, however, the first housing may be in the shape of a cylinder, i.e. having a circular or oval cross section. Alternatively, however, the first housing may have any other suitable shape. The shape as well as the cross section of the first housing may vary along the length of the first housing, i.e. from its distal to its proximal end. Preferably, the first housing is made of a polymer material. The polymer material preferably is a thermoplastic. The outer face of the first housing preferably comprises at least one area which is adapted to provide a more agreeable grip for a hand of a person, e.g. such an area may comprise a softer material and/or a shape conforming to fingers or a part of a human hand.

The first connection means serve to releasably couple the drive unit with a syringe unit. Hence, the first connection means are shaped, dimensioned and placed such as to enable a releasable coupling with the second connection means of the syringe unit. The syringe unit is hence releasably coupled to the proximal end of the drive unit with its distal end. I.e. the proximal end of the first housing of the drive unit is thereby releasably coupled to the distal end of the second housing of the syringe unit.

The actuator is preferably arranged to be movable along the central axis of the first housing by means of the linear drive. The linear drive preferably comprises an electric motor. Alternatively, the linear drive may comprise other drive means, such as e.g. a spring element which may be compressed or tensioned after or before coupling the syringe unit.

If the linear drive comprises an electric motor, then the electric motor preferably is a servo motor or a stepper motor. Use of a servo motor or a stepper motor allows to calculate, preferably in combination with an encoder the position of the actuator along the first central axis using the number of full rotations and/or the angular position of the shaft of the servo or stepper motor. The rotational movement of the electric motor is preferably converted to a linear movement by means of a gear. Preferably, the actuator is in the form of a nut which is engaged with a rod by means of threads, the rod being driven in rotation by the electric motor. The nut comprises means which prevent its rotation. Hence, when the rod is rotating, the nut will be moved along the rod in two directions, depending on the direction of rotation of the rod. Preferably, the rod is directly connected to the output of the electric motor. Alternatively, however, the rod may be coupled to the output of the electric motor by means of a gear arrangement, such as e.g. at least one spur wheel, a worm gear or the like. The rod is preferably arranged along the central axis of the first housing. The rod and the nut preferably are made of a polymer. Alternatively, the rod and/or the nut may be made of a metal or metal alloy.

In the case that the drive comprises an electric motor, the drive unit further comprises a source of electrical energy. Preferably, the source of electrical energy is in the form of at least one primary or secondary battery located in the drive unit. If the source of electrical energy is in the form of at least one primary battery, the drive unit comprises a battery holder with a removable cover, such that the at least one primary battery may be manually replaced with a fresh battery once its electrical charge is exhausted. In the case that the source of electrical energy is at least one secondary battery, the drive unit comprises a socket for an electrical cable in order to re-charge the at least one secondary battery. Preferably, said socket is in the form of a USB socket. The at least one secondary battery preferably is a lithium polymer battery or a lithium ion battery. In this case, the syringe unit preferably comprises a corresponding battery charging circuit. Alternatively, the at least one secondary battery may also be charged by means of inductive charging.

The first sleeve is preferably shaped and dimensioned such as to slide along an inner wall of the first housing when it is moved linearly. The first sleeve preferably is substantially hollow such that it does not interfere with other elements of the drive unit, for example the actuator and linear drive, when the first sleeve is linearly moved.

The first elastic element preferably is a spring element. The spring element preferably is a helical spring. Alternatively, the spring element may also be a spiral spring or be of any other suitable configurations, such as a gas strut, a piece of an elastomer or the like.

Preferably, the first elastic element is arranged between the first sleeve and a distal end of the first housing. In this case, the first elastic element preferably is compressed when the actuator is in the initial position, thus exerting a force onto the first sleeve pushing it towards the proximal end of the first housing.

The movement of the first sleeve in direction of the proximal end of the first housing is limited by the interaction of the first sleeve with the stop surface of the actuator. In order to interact with the stop surface of the actuator, the first sleeve preferably comprises an arm, protrusion or the like which may abut on the stop surface. Preferably, the first sleeve comprises a collar which extends from an inner surface of the first sleeve towards the actuator, said collar being sized and shaped such as to abut onto a stop surface of the actuator. If the actuator is a nut, said stop surface preferably is in the form of a circumferential ledge on an outside surface of the nut. The actuator may comprise one or more stop surfaces. Accordingly, the first sleeve is preferably configured to be able to abut onto all stop surfaces of the actuator.

It is to be noted that the first sleeve is free to move towards the distal end of the drive unit against the bias of the first elastic element. In this case, the first sleeve disengages from the stop surface of the actuator.

The longitudinal second housing preferably is generally in the shape of a cuboid, preferably with rounded edges. I.e. the housing has a rectangular cross section with rounded corners in this case. Alternatively, however, the first housing may be in the shape of a cylinder, i.e. having a circular or oval cross section. Alternatively, however, the first housing may have any other suitable shape. Preferably, the first housing is made of a polymer material. The polymer material preferably is a thermoplastic.

Preferably, the distal end of the second housing is partially inserted into the first housing at its proximal end when the syringe unit is coupled to the drive unit, such that a secure connection between the syringe unit and the drive unit is enabled.

The shape and size of the second housing at the distal end thereof or in an area which neighbors the proximal end of the first housing, in the case where at least a part of the second housing is inserted into the first housing, preferably essentially matches the shape and size of the first housing at its proximal end. Hence, when a syringe unit is coupled to the drive unit, the auto injector has a smooth, unitary outer shape.

When the syringe unit is coupled to the drive unit, the first length axis and the second length axis are congruent or parallel to each other. In order to couple the syringe unit to the drive unit, the first connection means arranged at the proximal end of the first housing are engaged with the second connection means arranged at the distal end of the second housing. Preferably, the first and second connection means are of the form-fit type, preferably of the snap-fit type. In this case the syringe unit may be removed manually from the drive unit by depressing the first connection means or the second connection means in order to allow a disengagement of the first and second connection means. The syringe preferably comprises a barrel defining a volume within which the medicament is stored. Further, the syringe includes a needle which is coupled to a first end of the barrel. A plunger is slidably arranged within the barrel, such that movement of the plunger towards the first end of the barrel expels the medicament through the needle. The syringe is arranged within the second housing of the syringe unit such that the needle points towards the proximal end of the second housing. Thereby, the tip of the needle may protrude out of the proximal end of the second housing.

The barrel of the syringe preferably has a volume of from 0.1 ml to 5.0 ml, more preferably of 1 ml to 2.25 ml. The needle preferably has an outer diameter of at least 0.3 mm and an inner diameter of at least 0.15 mm. Preferably, the needle is a 29 or 27 gauge needle or larger.

The cap is releasably coupled to the proximal end of the second housing of the syringe unit and closes off the interior space of the second housing. Further, the cap sheathes the tip or part of the needle such that unintentional pricking is avoided, especially in cases where the needle protrudes from the proximal end of the second housing. Further, the cap ensures that the needle is kept sterile prior to its use. Preferably, the cap may further comprise or engage with a rigid needle sheath which encloses the needle at least partially and ensures sterility of the latter.

The cap is releasably coupled to the proximal end of the second housing by means of an engagement of the third and the fourth connection means. The third and fourth connection means preferably are of the form fit type, more preferably of the snap fit type. In this case the cap may be removed manually by depressing the third connection means or the fourth connection means in order to allow a disengagement of the third and fourth connection means.

The second sleeve is preferably shaped and dimensioned such as to slide along an inner wall of the second housing when the second sleeve is moved linearly along the second length axis. The second sleeve preferably is substantially hollow such that it does not interfere with other elements of the syringe unit, for example the syringe.

Initially, the second sleeve is preferably positioned such that its distal end lies in the proximity of the distal end of the second housing. In this position, the second sleeve is configured to block the disengagement of the third connection means the fourth connection means.

When the actuator is moved in direction of the proximal end of the first housing from an initial position to a first position, the first sleeve is synchronously pushed along by means of the first elastic element. The first sleeve is configured such as to block a disengagement of the first connection means with the second connection means when the actuator is in the first position. In the embodiment where the first and second connection means are of the snap fit type, the first sleeve preferably blocks either the first or the second connection means to move or flex away of the second or first connection means, respectively. Hence, when the actuator is in the first position, the syringe unit and the drive unit are securely coupled together and may not be uncoupled unintentionally. For uncoupling the syringe unit, for example after the syringe has been emptied, the actuator is retracted to the initial position, dragging along the first sleeve in the distal direction against the bias of the first elastic element.

When the actuator is moved further towards the proximal end of the first housing to a second position, the first sleeve is further pushed by the first elastic element towards the proximal end of the first housing. When the actuator reaches a certain position between the first position and the second position, the proximal end of the first sleeve abuts onto the distal end of the second sleeve. Further movement of the actuator and of the first sleeve towards the proximal end of the first housing thereby leads to a movement of the second sleeve towards the proximal end of the second housing by the pushing action of the first sleeve. The first sleeve and the second sleeve are configured such that the blocking of the third and fourth connection means by the second sleeve is released when the actuator reaches the second position. In this case, the cap may be manually removed.

In the case that the third and the fourth connection means are configured as snap fit connection, the second sleeve is preferably configured to block a deflection of the third or fourth connection means from the fourth or third connection means, respectively.

Preferably, a proximal end of the second sleeve is configured to move into the cap to a first active position when the second sleeve is pushed towards the proximal end of the second housing by the first sleeve.

In this embodiment, the proximal end of the second sleeve thus may act to shield the needle to offer a pricking protection when the cap is removed. Hence, safety of the auto injector may be further increased. Of course, in order to act as needle shield, the second sleeve has to be preferably configured to completely sheath the needle when the second sleeve is in the first active position. When the needle is inserted into the target tissue, the second sleeve is moved distally against the bias of the first elastic element, to reveal the needle.

Preferably, the cap comprises a second elastic element which is compressed or is arranged to be compressed when the proximal end of the second sleeve moves into the cap towards the first active position.

The second elastic element preferably is in the form of a spring, especially of a helical spring. Alternatively, the second elastic element may also be configured as a piece of elastomeric material or the like. The second elastic element exerts a force which is directed towards the distal end of the syringe unit when said second elastic element is compressed by the movement of the second sleeve into the cap. Hence, the force of the second elastic element counteracts the force of the first elastic element, as both elastic elements interact via the first sleeve and the second sleeve. When the second sleeve is at the first active position it abuts on a stop surface preventing any further movement of the second sleeve into the cap.

Preferably, the first sleeve, the second sleeve and the actuator are dimensioned such that the first sleeve is spaced apart from the stop surface when the proximal end of the second sleeve is in the first active position, and configured to be pushed into abutment with the stop surface by means of the first elastic element when the cap is removed, thus allowing the proximal end of the second sleeve to be pushed into a second active position. To this end, proximal movement of the second and first sleeve, under the bias of the first elastic element, is halted by means of the second elastic element well before the actuator reaches the second position.

When the cap is removed the second elastic element is removed also, so that the second sleeve and the first sleeve are pushed in the proximal direction by means of the first elastic element. When the first sleeve abuts the stop surface and/or when the second sleeve abuts an abutment element of the syringe unit the movement of the first and second sleeve is stopped and the proximal end of the second sleeve is in the second active position.

Movement of the proximal end of the second sleeve into the second active position may be used to detect that the auto injector is in a status ready for pricking the skin and starting of the injection.

Preferably, the second sleeve comprises locking elements which prevent movement of the second sleeve when the distal end of the second sleeve is spaced apart from the proximal end of the first sleeve and which allow movement of the second sleeve when the proximal end of the first sleeve abuts on the distal end of the second sleeve.

Hence, the second sleeve is secured from moving within the second housing before the actuator is moved from the first position towards the second position. Further, if the injection of the medicament is aborted, i.e. by the patient or medical personnel, the actuator is preferably retracted to the initial position and the second sleeve is again locked in position within the second housing.

Preferably, the locking means are in the form of at least one locking hook which engages an inner wall of the second housing, preferably into at least one corresponding first notch provided in the inner wall, such as to prevent the second sleeve from moving. In this case the first sleeve comprises interference means at its proximal end which flex the at least one locking hook away of the inner wall of the second housing or out of the at least one first notch when the proximal end of the first sleeve abuts on the distal end of the second sleeve.

The second sleeve preferably comprises fifth connection means at a distal end. Said fifth connection means are configured to engage with sixth connection means of a follower element. The follower element is arranged linearly movable along the second central axis in the second housing. The fifth connection elements engage the sixth connection elements when said second sleeve is moved to a retracted position against the bias of the first elastic element when the auto injector is pressed onto skin of a patient. This movement of the second sleeve into the retracted positon unsheathes the needle for pricking the skin.

The second sleeve is moved from the second active position in a distal direction towards the retracted position. This movement is preferably caused by positioning the proximal end of the second sleeve on the skin of a patient and pressing the auto injector towards the skin. By pressing the auto injector towards the skin, the second sleeve is gradually moved into the retracted position such that the needle may prick and gradually penetrate the skin and tissue of the patient. The proximal end of the second sleeve thereby remains in contact with the skin, still sheathing the part of the needle which is not inserted into the skin and tissue of the patient. As the distal end of the second sleeve abuts on the proximal end of the first sleeve, the first sleeve is also moved in the distal direction against the biasing force of the first elastic element. Hence, the movement of the second sleeve towards the retracted position occurs against the biasing force of the first elastic element. Therefore, the first elastic element will push the first sleeve and also the second sleeve back in the proximal direction, when the auto injector is moved away of the skin until the proximal end of the second sleeve reaches the second active position again. This ensures that the needle always remains sheathed when it is not inserted into the skin and tissue of the patient.

The fifth and the sixth connection means preferably are of the form-fit type, especially of the snap-fit type, which engage with each other when the second sleeve reaches the retracted position.

Movement of the second sleeve into the retracted position signals that the needle is completely inserted into the skin of the patient and that the medicament may be injected.

Preferably, the follower element comprises hook elements which are configured to engage into a permanent form-fit connection with retainer elements arranged on an inner wall of the second housing when the second sleeve is moved from the retracted position to the second active position by means of the first elastic element, e.g. when the auto injector is removed from the skin. Therefore, the needle is permanently sheathed once the syringe unit has been used, thus preventing an accidental re-use of the syringe unit or an accidental pricking with a used needle. This increases the safety of the auto injector.

As the follower element is coupled to the second sleeve by means of the fifth and sixth connection elements, the follower element is moved proximally together with the second sleeve. The second sleeve itself is moved by means of the biasing force of the first elastic element back towards the second active position, e.g. once the auto injector is removed from the skin of the patient. Preferably, the actuator is configured to abut on a plunger of the syringe in the second position. Hence, if the actuator is subsequently moved further towards the proximal end of the first housing, the actuator will move the plunger of the syringe such that a medicament filled within the barrel is expelled through the needle.

The drive unit preferably comprises an electronic control module as well as at least one detector which detects when the first sleeve reaches at least one defined position along the first central axis and/or whether the first connection means are engaged with the second connection means, the at least one detector preferably being a dome tact switch or a switch actuator.

The electronic control module preferably comprises a microcontroller or microprocessor which controls the linear drive, preferably depending on signals provided by the at least one detector.

Preferably, the drive unit comprises several detectors, of which a first detector detects whether the first connection means are engaged with the second connection means and the remaining detectors are configured to detect when the first sleeve or the actuator reaches at least one defined position, such as e.g. the first position and/or the second position.

The electronic control module is preferably configured such as to move the actuator from the initial position to the first position when a first detector detects that the first connection means are engaged with the second connection means. In the case that the first and the second connection means are in the form of a form-fit or snap-fit element, the first detector preferably is a dome tact switch which is depressed when the first and second connection means engage.

Preferably, the drive unit comprises a second detector which is positioned such as to detect when the first sleeve is brought into connection with the stop surface after the cap is removed, i.e. when the proximal end of the second sleeve is pushed into the second active position. This signals that the auto injector is in a configuration ready for the injection.

Preferably, the electronic control module is configured such that the actuator is fully retracted to the initial position if the injection of the medicament is aborted. Abortion of the injection may be signaled by the patient or medical personnel by pressing a corresponding button or the like on the drive unit, or the electronic control module may be configured to abort the injection if the second sleeve is not moved into the retracted position for a pre-specified time period after the proximal end of the second sleeve is pushed into the second active position.

If the cap has not been removed when the injection is aborted, the second sleeve will be pushed distally by the second elastic element of the cap when the actuator is retracted to the initial position. Hence, in this case, the disengagement of the second sleeve and the first sleeve occurs once the second sleeve reaches the distal end of the second housing. Therefore, if the second sleeve comprises locking elements, the locking elements may re-engage with the inner wall of the second housing or in the at least one first notch.

As the second sleeve is moved distally, the second sleeve will again block the disengagement of the third and fourth connection means, thus locking the cap again to the proximal end of the second housing. Thus, unintentional removal of the cap with the associated risk of pricking is avoided.

If the cap has been removed prior to the abortion of the injection, the proximal end of the second sleeve will remain in the second active position when the actuator is retracted to the initial position. If the second sleeve comprises locking elements, these locking elements will therefore engage the inner wall of the second housing in this case. Preferably, the second housing comprises at least one second notch on the inner wall which is positioned such that the locking elements may engage with said at least one second notch when the proximal end of the second sleeve is in the second active position and the actuator is retracted. In this embodiment, the second sleeve will hence remain permanently locked with its proximal end in the second active position upon abortion of the injection, thus sheathing the needle. By sheathing the needle the risk of unintentional pricking is avoided.

The electronic control module is preferably configured to move the actuator from the second position further proximally in order to push the plunger of the syringe so that a medicament present in the barrel of the syringe is expelled through the needle and injected into a patient. Preferably, the electronic control module is configured such as to move the actuator from the second position proximally along a defined distance, said distance corresponding to the ejection of a defined volume of the medicament in the barrel of the syringe. Preferably, the syringe unit comprises means to detect the type of syringe and/or the volume of the syringe arranged in the syringe unit as well as input means which allow the selection of the volume of medicament to be injected. The electronic control module may in this case calculate the defined distance the actuator needs to be moved proximally from the second position in order to expel the selected volume of the medicament based on the detected syringe type or syringe volume.

This movement of the actuator from the second position in the proximal direction is started by the electronic control module upon receipt of a signal. This signal may be generated by input means, e.g. such as a button, which are activated by a patient or medical personnel once the skin of the patient is pricked with the needle.

However, preferably, the syringe unit comprises a third detector which is arranged to detect a position of the first sleeve which indicates that the second sleeve has reached the retracted position. Bear in mind that the distal end of the second sleeve abuts on the proximal end of the first sleeve, such that a movement of the second sleeve in the distal direction leads to a likewise movement of the first sleeve in the distal direction. The third detector is thereby configured to send the signal to the electronic control unit to start the movement of the actuator from the second position in the proximal direction. The third detector preferably is a dome tact switch which is depressed by the first sleeve when the second sleeve reaches the retracted position.

The electronic control module preferably is further configured such that when the third detector detects that the first sleeve leaves the position which indicates that the second sleeve is in the retracted position the actuator is moved distally back to the initial position. Preferably, this movement is delayed for a pre-defined time, such as to allow the second sleeve to be pushed back such that its proximal end reaches the second active position by means of the first elastic element.

In a preferred embodiment, the second sleeve is coupled to the follower element by means of the fifth and sixth connection elements. Once the proximal end of the second sleeve reaches the second active position, the hook elements of the follower element will engage with the retainer elements thus permanently locking the second sleeve within the second housing.

In other embodiments where no follower element is present, the second sleeve is preferably locked with its proximal end in the second active position by means of the locking elements.

Preferably, the electronic control module comprises at least one wireless communication module, such as a Bluetooth^{®} or WiFi module. Alternatively, the at least one wireless communication module may be a module capable to connect to a cellular network, such as a 4G or 5G communication module. The electronic control module preferably is configured to exchange data with an external device, such as a mobile device or server. The mobile device may be a cellular phone, tablet computer, notebook, personal computer, smartwatch or personal digital assistant (PDA). Hence, in this embodiment, a patient or a doctor may keep track of the injections administered with a specific auto injector, for example to ensure that a treatment plan is followed.

Preferably, the electronic control module comprises means to identify the syringe unit coupled to the drive unit. This allows the electronic control unit to acquire information about certain parameters of the syringe unit, such as charge number, product number, manufacturing date, type of the syringe, volume of the syringe, medicament stored in the barrel of the syringe and/or expiry date of the medicament.

The means for identification may be of any suitable type. For example, the drive unit may comprise a barcode reader which is able to read a linear or matrix barcode attached to or printed on a specific location of the syringe unit. Alternatively, the means to identify the syringe unit may also comprise contact pins which may establish an electric contact with a chip of the syringe unit allowing the electronic control module to query data stored on the chip.

Preferably, however, the means to identify the syringe unit is the provision of an RFID reader in the drive unit. In this embodiment, the syringe unit comprises an RFID tag, preferably a passive RFID tag. Preferably, the RFID reader is an active reader, especially a near field communication (NFC) module. Hence, the auto injector comprises an active reader passive tag RFID or NFC system. Preferably, the active reader is further configured to be able to write data onto a passive RFID tag of the syringe unit. This allows to tag a syringe unit as used after the injection or in the case that the injection is aborted.

The electronic control module is preferably configured such as to initiate a query with the means of identification once the at least one detector, especially the first detector, indicates that first and second connection means have engaged, i.e. that a syringe unit has been coupled to the drive unit.

Preferably, the electronic control module is further configured to check whether the expiration date of the medicament has not yet passed based on the charge number or expiry date and/or whether the syringe unit is compatible with the drive unit, e.g. based on the product number, type of the syringe and/or volume of the syringe. If the electronic control module comprises at least one wireless communication module, a database on a remote server may be queried by the electronic control module in order to acquire additional information on the syringe unit, e.g. based on a charge or serial number of the syringe unit. Hence, it is possible to ensure that only genuine syringe units or syringe units from a charge that has passed all quality and safety tests are used for an injection.

Further, the electronic control module is configured to check whether the syringe unit has not been tagged as used. In the case that the medicament is expired, the syringe unit is not compatible or the syringe unit has been tagged as used, the electronic control module is configured to not move the actuator from the initial position to the first position.

Preferably, the drive unit comprises a temperature sensor which measures the temperature, preferably of the syringe. The electronic control module preferably is configured not to move the actuator from the initial position or to abort the injection if the measured temperature is outside of a pre-defined range. The pre-defined range varies for different medicaments and may hence be determined by the electronic control module based on the information acquired by the means to identify the syringe unit.

Preferably, the drive unit comprises output means to signal a status of the auto injector to a patient, the output means preferably being a screen, at least one light indicator, a vibration generator and/or an audio signal generator.

The output means preferably indicate the status of the auto injector based on signals of the at least one detector and/or the position of the actuator. Hence, the output means may indicate the coupling of the syringe unit with the drive unit, the blocking of the first and second connection means by the first sleeve, the unblocking of the third and fourth connection means by the second sleeve for cap removal, start and end of the medicament injection and/or release of the first and second connection means.

If the output means comprises a screen, the screen preferably is a liquid crystal display or an electronic paper display. Preferably, the electronic control module is configured to additionally display information about the syringe unit on the screen, for example according to information acquired by the means to identify the syringe unit. Preferably, the screen may also show the next step to be carried out by the patient or medical personnel using the auto injector. On the other hand, the output means may explicitly exclude a screen or display, in particular in case elaborate graphical patient guidance is offered by a mobile device of the patient.

The output means may comprise at least one light indicator, preferably in the form of an LED. More preferably, the output means comprises several light indicators, preferably of different colors. The status of the auto injector may be signaled by means of switching specific light indicators on or off by the electronic control module.

The output means may also comprise a vibration generator. In this embodiment, the electronic control module is preferably configured to generate different vibration patterns in order to indicate different status of the auto injector to the patient or medical personnel.

The output means may also comprise an audio signal generator. In this case, the electronic control module is configured to generate different audio signals to indicate the different status of the auto injector.

Other advantageous embodiments and combinations of features come out from the detailed description below and the entirety of the claims.

### Brief description of the drawings

The drawings used to explain the embodiments show:
- Fig. 1: an exemplary embodiment of an auto injector according to the present invention;
- Fig. 2: an explosion view of the drive unit of the embodiment according to Fig. 1;
- Fig. 3: an explosion view of the syringe unit of the embodiment according to Fig. 1;
- Fig. 4, 5: a length-section of the auto injector according to the embodiment as shown in Fig. 1 in an initial state;
- Fig. 6, 7: the auto injector according to Figures 4 and 5 in a state where the actuator is in the first position;
- Fig. 8, 9: the auto injector according to the Figures 6 and 7 in a state where the actuator is in the second position;
- Fig. 10, 11: the auto injector according to Figures 8 and 9 after the cap has been removed;
- Fig. 12, 13: the auto injector according to Figures 10 and 11 in the situation where the needle has pricked the skin of a patient;
- Fig. 14, 15: the situation after the injection has been terminated and the auto injector has been removed from the skin of the patient;
- Fig. 16 - 18: length sections of the syringe unit in different situations where the movement of the second sleeve is blocked.

In the figures, the same components are given the same reference symbols.

### Preferred embodiments

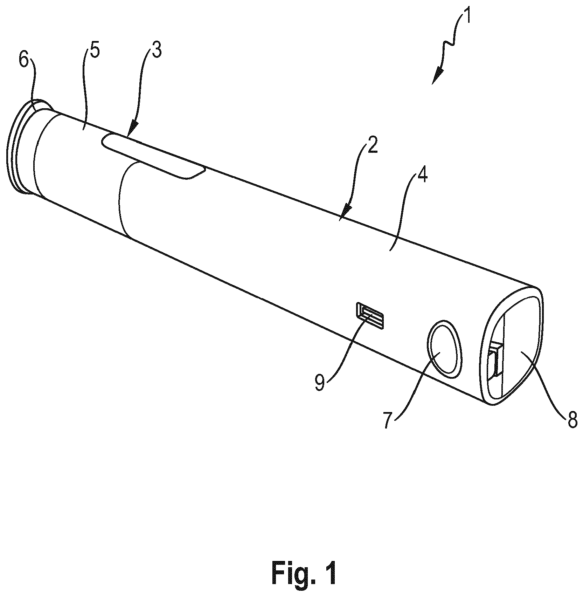

Fig. 1 shows an embodiment of an auto injector 1 according to the present invention in an isometric view. The auto injector 1 comprises a drive unit 2 with a longitudinal first housing 4. At the proximal end of the first housing 4 a syringe unit 3 with a longitudinal second housing 5 is coupled to the drive unit 2. A cap 6 is coupled to the proximal end of the second housing 5. In the embodiment as shown, the drive unit 2 comprises a button 7 to operate the auto injector 1 as well as a screen 8 which is arranged at a distal end of the first housing 4. Further, a plug 9 for a USB-C charging cable is arranged on the drive unit 2 in the embodiment as shown.

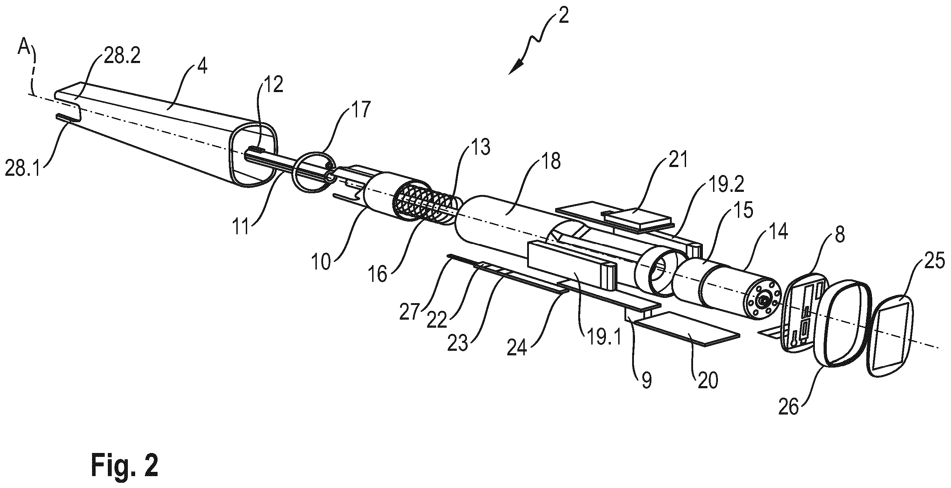

Fig. 2 shows an explosion view of the drive unit 2 of the embodiment of the auto injector 1 according to Fig 1. The drive unit 2 comprises the longitudinal first housing 4. The first housing 4 has a first central axis A. On the figure, the proximal end of the first housing 4 is on the left and the distal end accordingly on the right side. At the proximal end, the first housing 4 comprises two opposed lugs 28.1, 28.2 which protrude proximally.

Within the first housing 4, an actuator 11 is arranged on said first central axis A. The actuator 11 is movable along the first central axis A by means of a linear drive. The linear drive includes an electric motor 14 which drives a threaded shaft 13 in rotation by means of a gear 15. By interaction of the threaded shaft 13 with internal threads of the actuator 11 the latter may be moved linearly about the first central axis A. Depending on the rotation direction of the threaded shaft 13, the actuator may be moved proximally or distally along the first central axis A. Further, a first sleeve 10 is arranged within the first housing 4, the first sleeve 10 being movable longitudinally along the first central axis A within the first housing 4. The first sleeve 10 is biased towards the proximal end of the first housing 4 by means of a first elastic element 16, which is embodied as coil spring in the embodiment as shown. A stop surface 12 which is arranged on a proximal portion of the actuator 11 limits the movement of the first sleeve in the proximal direction.

The electric motor 14, the gear 15, the threaded rod 13, the first sleeve 10, the first elastic element 16 and the actuator 10 are arranged within a holder 18 within the first housing 4. The holder 18 is prevented from moving proximally by means of a spring lock washer 17 arranged within the first housing 4.

Between the holder 18 and an inner wall of the first housing 4 the drive unit comprises an electronic control module 20 as well as a wireless communication module 21. The wireless communication module 21 enables the exchange of data with further devices by means of a wireless transmission, such as Bluetooth^{®}, WiFi or by means of a cellular network. The electronic control module 20 includes a circuit board on which the necessary electronic components for the control of the electric motor 14 as well as of the screen 8 are arranged. Further, the circuit board of the electronic control module 20 includes the plug 9 with which two secondary batteries 19.1, 19.2, which are preferably lithium polymer batteries, may be charged.

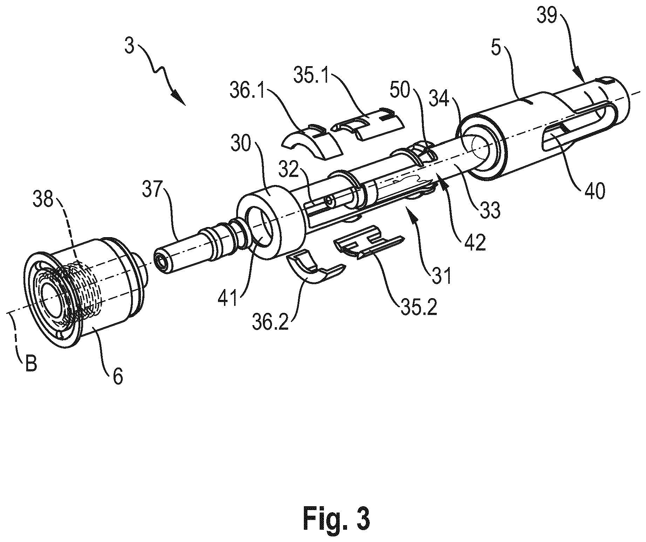

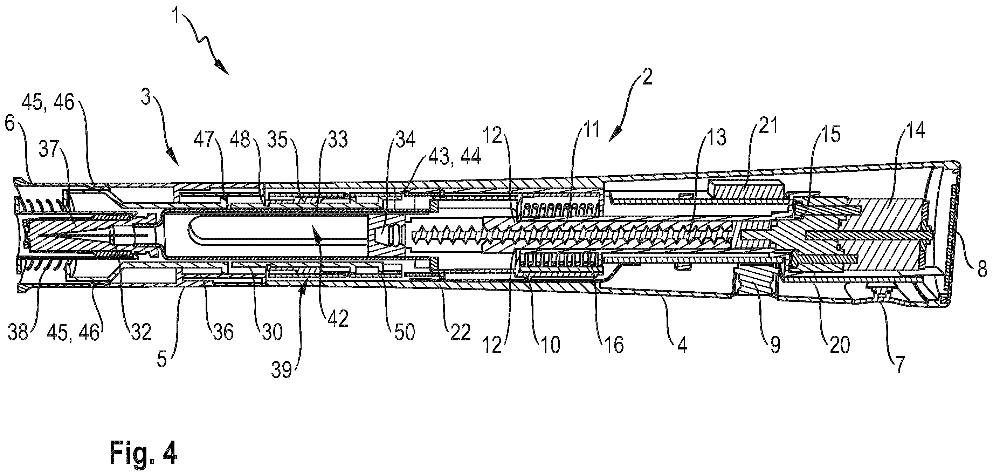

Further, a first detector 22, a second detector 23 and a third detector 24, all in the form of dome tact switches in the embodiment as shown, are arranged on the circuit board of the electronic control module 20. It is understood that the three detectors 22, 23, 24 may alternatively not be directly attached to the circuit board, but rather to other elements of the drive unit 2, such as e.g. the inner wall of the first housing 4 or on the holder 18, and connected to the electronic control module 20 by means of wires. The first detector 22 serves the purpose to detect whether first connection means 43 of the drive unit are engaged with second connection means 44 of the syringe unit (see Fig. 4). The second detector 23 and the third detector 24 detect when the first sleeve 10 has reached specific positions along the central axis A. The drive unit 2 also comprises a thermal element 27 which is arranged such that it may measure the temperature of a medicament in the syringe unit 2 when the syringe unit is connected with the drive unit 2 (see Fig. 3). To this end, the thermal element 27 is arranged to lie on the inside of one of the lugs 28.1, 28.2. The screen 8 at the distal end of the first housing 4 is arranged in a frame 26 and covered by a protective glass 25.

Fig. 3 shows an explosion view of the syringe unit 3 of the embodiment of the auto injector 1 according to Fig. 1. The syringe unit 3 comprises a longitudinal second housing 5 with a second central axis B. When the syringe unit 3 is coupled with the drive unit 2, the first central axis A of the drive unit 2 and the second central axis B of the syringe unit 3 are congruent, i.e. the first central axis A and the second central axis B form an imaginary, single straight line. The second housing 5 includes a syringe 31. The syringe 31 comprises a barrel 33 which encloses a volume 42 into which a medicament may be filled. The syringe 31 further comprises a hollow needle 32 at its proximal end to prick the skin of a patient in order to administer the medicament to the patient. A plunger 34 is arranged in the barrel 33 at its distal end. By moving the plunger 34 within the barrel 33 in the proximal direction, the medicament filled in the volume 42 may be expelled through the needle 32. A second sleeve 30 is arranged within the second housing 5, the second sleeve 30 being linearly movable along the second central axis B within the second housing 5. The second sleeve 30 is concentric with the barrel 33 of the syringe 31. At its proximal end, the second sleeve 30 includes an opening 41, through which the needle 32 protrudes. At its distal end, the second sleeve comprises at least one locking element 50 which may engage with the inner wall of the second housing 5 or with notches provided in said inner wall to prevent the second sleeve 30 from moving within the second housing 5.

The second housing 5 further includes a follower element 35, which is made of two separate parts 35.1, 35.2 in the embodiment as shown. The follower element 35 is linearly movable along the second length axis B about the second sleeve 30. Further, the second housing 5 includes an abutment element 36, which is shown in two pieces 36.1, 36.2 in the figure. At the proximal end, the cap 6 is connected to the second housing 5. The cap 5 comprises a rigid needle sheath 37 as well as a second elastic element 38, which is configured as spiral spring in the embodiment as shown. Towards the distal end, the second housing 5 comprises a distal section 39 with a reduced outer diameter, the distal section 39 being insertable into the first housing 4 of the drive unit 2. The second housing 5 comprises two lateral protrusions 40, into which the lugs 28.1, 28.2 of the first housing 4 are inserted when the syringe unit 3 is coupled to the drive unit 2.

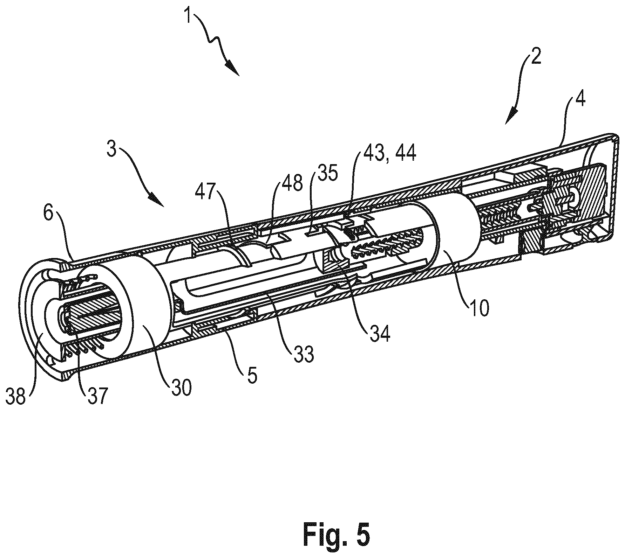

Fig. 4 shows a length-section of the auto injector 1 according to the embodiment as shown in Fig. 1 in an initial state when the syringe unit 3 has just been coupled to the drive unit 2. Fig. 5 shows the same length section in a partial isometric view. As may be seen on Fig. 4, the distal section 39 of the second housing 5 is inserted into the proximal end of the first housing 4.

The distal end of the second housing 5 of the syringe unit 3 is connected to the proximal end of the first housing 4 of the drive unit 2 by engagement of first connection means 43 of the first housing 4 with second connection means 44 of the second housing 5. In the embodiment shown, the first connection means 43 and the second connection means 44 are in the form of complementary snap-fit elements. It is to be noted that while two pairs of first connection means 43 and second connection means 44 are depicted in Fig. 4, the auto injector 1 may include more such pairs. However, provision of two pairs of first connection means 43 and second connection means 44 allows an easy removal of the syringe unit 3 from the drive unit 2 as the second housing 5 may simply be compressed on two opposing sides to unlatch the second connection means 44 from the first connection means 43. Engagement of the first connection means 43 with the second connection means 44 is detected by the first detector 22.

The actuator 11 is in the initial position which corresponds to its most distal position in the embodiment as shown. As the first sleeve 10 abuts on the stop surface 12 of the actuator 11, the first sleeve 10 is prevented from being moved proximally by means of the biasing force of the first elastic element 16. The first sleeve 10 is in its most distal position. The first elastic element 16 is in a compressed, i.e. energized state. The first sleeve 10 is spaced apart from the pairings of first connection means 43 and second connection means 44, so that the connection means 43, 44 may be engaged and disengaged.

The second sleeve 30 is held in a distal position by means of the locking means 50 which are in engagement with the inner wall of the second housing 5. The cap 6 is connected to the proximal end of the second housing 5 by means of an interaction between third connection means 45 and fourth connection means 46. The third connection means 45 and fourth connection means 46 are configured as complementary snap-fit elements. The second sleeve 30 extends proximally so far as to block the disengagement of the third connection means 45 with the fourth connection means 46 when it is in the distal position. In essence, the second sleeve 30 is sized such as to prevent a deflection of the third connection means 45 from the fourth connection means 46. The proximal end of the second sleeve thereby loosely abuts on the second elastic element 38 of the cap 6.

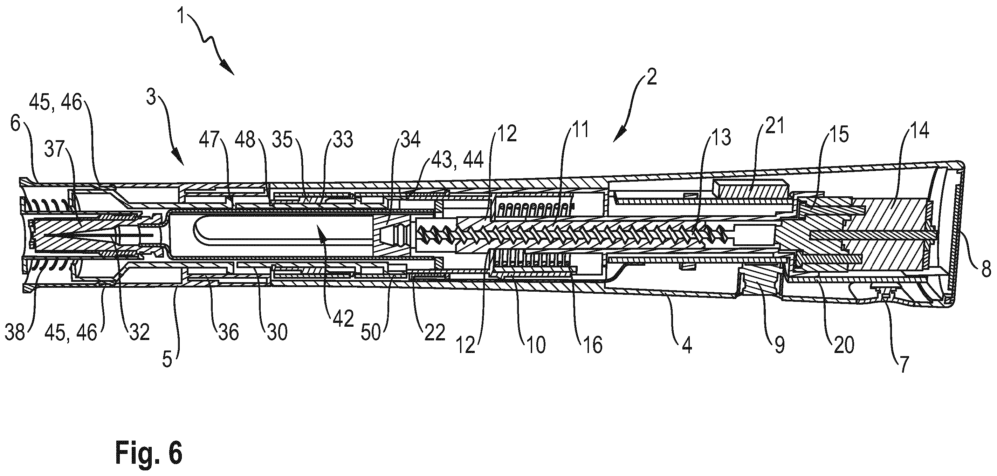

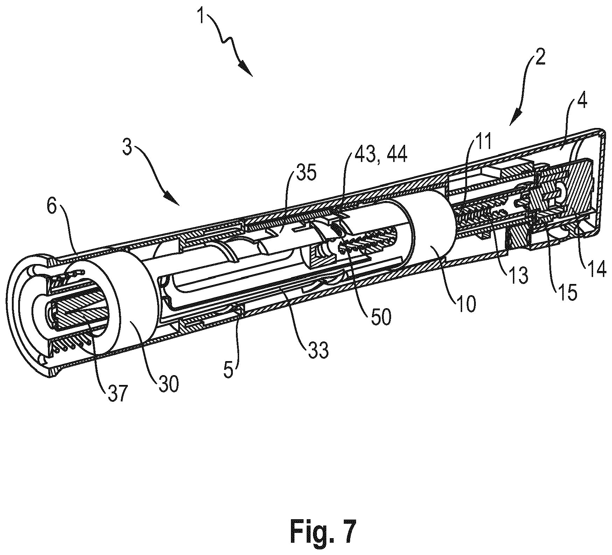

Figures 6 and 7 show the auto injector 1 according to Figures 4 and 5 in a state where the actuator 11 is in the first position. The actuator 11 is moved from the initial position to the first position as shown by rotational movement of the threaded rod 13. The threaded rod 13 is driven in rotation by means of the electric motor 14 and gear 15. The movement of the actuator 11 is started by the electronic control module 20 upon detection of an engagement of the first connection means 43 with the second connection means 44 by the first detector 22. The electric motor 14 is a step motor with which the electronic control module 20 is able to very precisely determine the distance that the actuator 11 is being moved, such that the actuator 11 may be very accurately moved from the initial positon to the first position.

As the first sleeve 10 is being pushed towards the stop surface 12 of the actuator 11 by means of the biasing force of the first elastic element 16, the first sleeve 10 moves in the proximal direction to the same degree as the actuator 11. When the actuator 11 is in the first position, the first sleeve 10 blocks the disengagement of the first connection means 43 and the second connection means 44. In the embodiment shown, the fist sleeve 10 blocks a deflection of the second connection means 44 of the second housing 5 from the first connection means 43 of the first housing 4. The locking elements 50 of the second sleeve are configured such as to be deflected from the inner wall of the second housing 5 or of notches in said inner wall when actuator 11 reaches the first position by means of an interaction with the proximal end of the first sleeve 10 with said locking elements 50. Hence, moving the actuator 11 and concurrently the first sleeve 10 not only securely locks the syringe unit 3 to the drive unit 2 but further also unblocks the second sleeve 30 such that the latter may move linearly within the second housing 5.

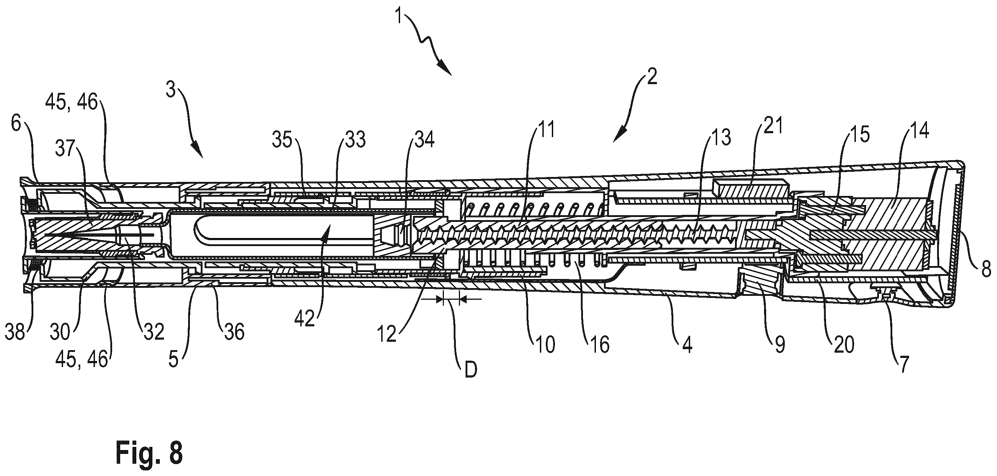

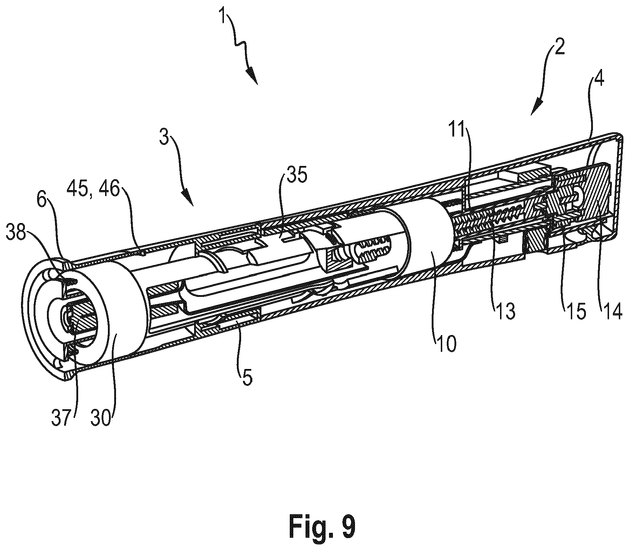

Figures 8 and 9 show the auto injector according to the Figures 6 and 7 in a state where the actuator 11 is in the second position, i.e. where the actuator 11 has been moved proximally from the fist position to the second positon by further rotation of the threaded rod 13 by means of the electric motor 14 and the gear 15. During the movement of the actuator 11 from the first position to the second position the proximal end of the first sleeve 10 abuts on the distal end of the second sleeve 30 and subsequently pushes the second sleeve 30 proximally. The proximal end of the second sleeve 30 is thereby pushed into the cap 6, thereby compressing the second elastic element 38. Once an equilibrium is reached between the biasing force of the first elastic element 16 and the biasing force of the second elastic element 38, the movement of the first sleeve 10 and of the second sleeve 30 is stopped. The proximal end of the second sleeve 30 is now in a first active position. The first elastic element 16 and the second elastic element 38 are configured such that the equilibrium is reached before the actuator 11 reaches the second position. This means that when the actuator 11 is in the second positon, the first sleeve 10 is spaced apart by a distance D from the stop surface 12. By moving the second sleeve 30 in the proximal direction, the third connection means 45 and the fourth connection means 46 are no longer blocked, such that the cap 6 may be removed from the proximal end of the first housing 5 by a patient or medical personnel. In the second position, the actuator 11 abuts on the plunger 34.

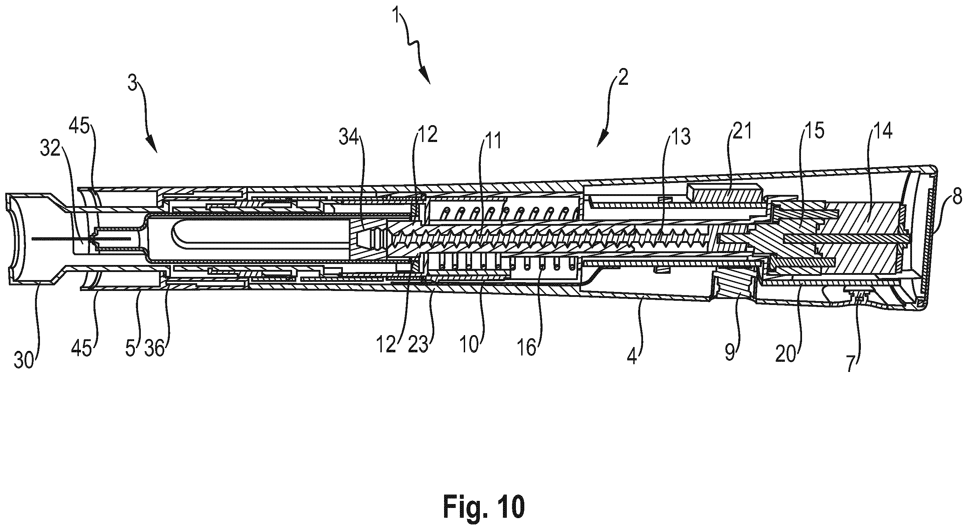

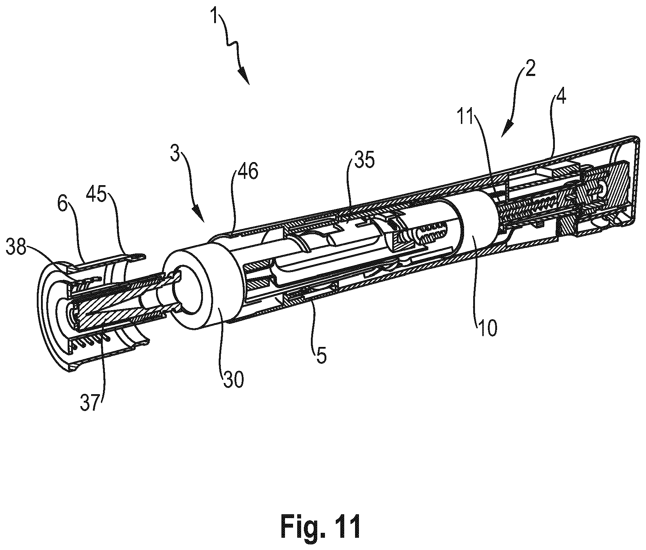

Figures 10 and 11 show the auto injector 1 according to Figures 8 and 9 after the cap 6 has been removed. As the cap 6 comprises the rigid needle sheath 37 and the second elastic element 38, these two elements are also removed together with the cap 6. However, by removing the second elastic element 38 the biasing force exerted by said second elastic element 38 falls away. Consequently, the first sleeve 10 is pushed proximally by means of the biasing force of the first elastic element 16 until the first sleeve 10 abuts again on the stop surface 12. As the proximal end of the first sleeve 12 abuts on the distal end of the second sleeve 30, said second sleeve 30 is moved further proximally as well until its proximal end reaches a second active position. The second sleeve 30 abuts on the abutment element 36 when the proximal end of the second sleeve 30 is in the second active position. I.e. any further proximal movement of the second sleeve 30 is prevented by the interaction between the abutment element 36 and the second sleeve 30. The second sleeve 30 is sized such that the needle 32 is completely sheathed by the second sleeve 30 when the proximal end of the second sleeve 30 is in the second active position. Thus, after removal of the cap 6 and of the rigid needle seal 37, the second sleeve 30 acts as needle shield. The second detector 23 is arranged such as to detect the proximal movement of the first sleeve 10 after removal of the cap 6, especially when the first sleeve 10 has reached a position where it abuts on the stop surface 12 again. The proximal movement of the first sleeve 10 into abutment with the stop surface 12 signals to the electronic control module 20 that the auto injector 1 is now ready to start the injection.

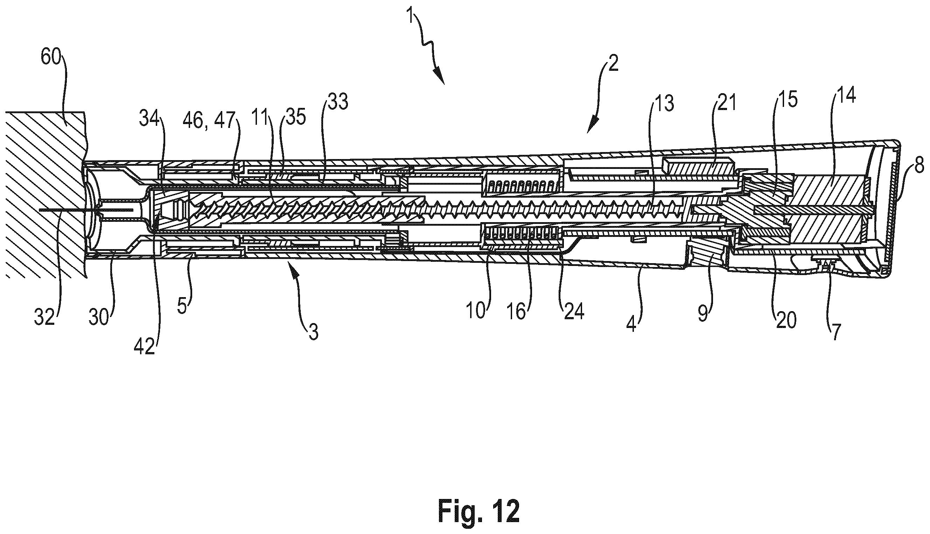

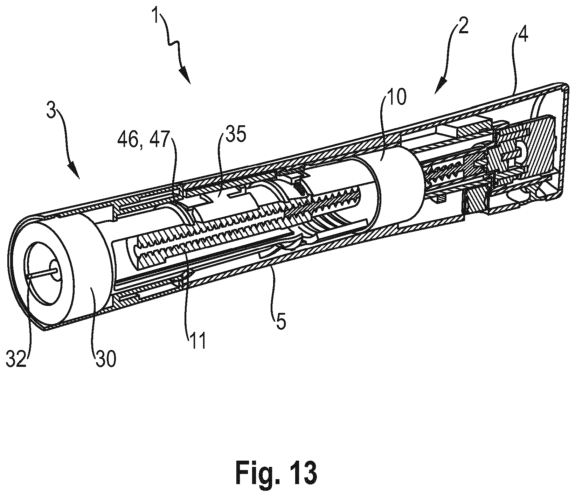

Figures 12 and 13 show the situation where the needle 32 has pricked the skin 60 of a patient. In order to prick the skin 60, the proximal end of the second sleeve 30 with the opening 41 is placed on the surface of the skin 60. The auto injector 1 is subsequently moved towards the skin 60. Through this movement, the second sleeve 30 is pushed distally to a retracted positon as shown and the needle 32 pricks the skin 60. The distal movement of the second sleeve 30 leads also to a distal movement of the first sleeve 10 and hence the first elastic element 16 is compressed. The third detector 24 detects when the first sleeve 10 reaches a position which corresponds to a retracted position of the second sleeve 30. This signals the electronic control module 20 that the needle 32 has reached its maximal penetration depth and that the injection with the medicament may start. Subsequently, the actuator 11 is moved further proximally, thereby moving the plunger 34 through the volume 42 of the barrel 33. This movement of the plunger 34 expels the medicament through the needle 32, such that the medicament is injected into the tissue of the patient. Figures 12 and 13 show the situation where the actuator 11 and the plunger 34 have reached their maximal proximal position and hence where the entire medicament has been injected into the tissue of the patient. When the plunger 34 and the actuator have reached this position, the electronic control module 20 displays a message on the screen 8 or issues another output, e.g. an audio signal, a vibration and/or a light indication to signal to the patient or medical personnel that the injection is complete and the auto injector 1 may be removed. Please note that the skin 60 is not shown on Fig. 13 in order not to mask any of the components of the auto injector 1.

The movement of the second sleeve 30 in the distal direction to the retracted position brings fifth connection means 46 of the second sleeve 30 into locking contact with sixth connection means 47 of the follower element 35, thereby permanently connecting the follower element 35 with the second sleeve 30.

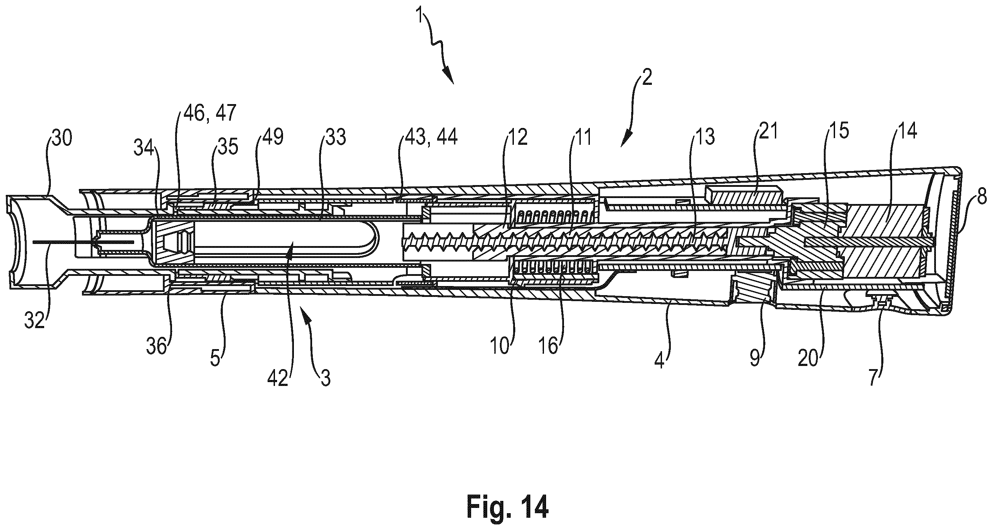

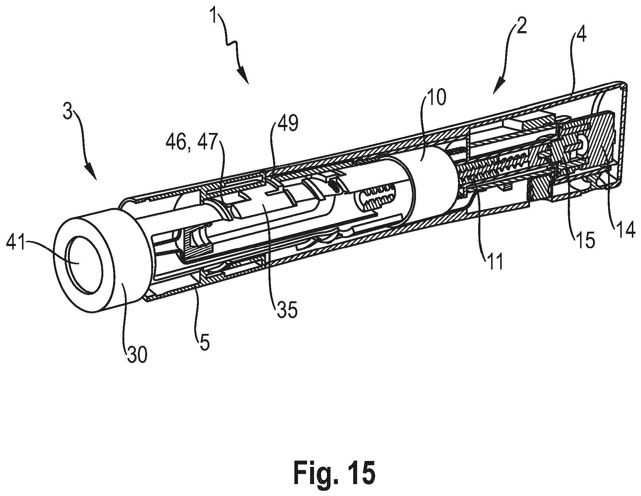

Figures 14 and 15 show the situation after the injection has been terminated and the auto injector 1 has been removed from the skin 60 of the patient. The first sleeve 10 and the second sleeve 30 are pushed proximally by the biasing force of the first elastic element 16 until the second sleeve 30 abuts on the abutment element 36 and the proximal end of the second sleeve 30 is again in the second active position. As the follower element 35 is connected to the second sleeve 30, the follower element 35 is moved in the proximal direction as well. When the second sleeve 30 abuts on the abutment element 36, hook elements 49 of the follower element 35 engage in matching recesses on the inner wall of the second housing 5, thereby permanently securing the second sleeve 5 within the second housing 5, i.e. after the hook elements 49 have engaged the recesses, any movement of the second sleeve 30 within the second housing 5 is prevented. The needle 32 is hence permanently sheathed by the second sleeve 30, hence avoiding any unintentional pricking of a person by the needle 32 or an unintentional re-use of the empty syringe unit 3.

The actuator 11 is moved distally back into the initial position by rotation of the threaded rod 13. The stop surface 12 will eventually abut onto the first sleeve 10. Subsequently, the first sleeve 10 will be dragged distally by the movement of the actuator 11 against the biasing force of the first elastic element 16. Distal movement of the first sleeve 16 unblocks the first connection means 43 and the second connection means 44, hence allowing a disengagement of the connection means 42, 43 in order to remove the syringe unit 3 from the drive unit 2.

After the actuator 11 has reached the initial position again and the syringe unit 2 is removed, the drive unit 2 is ready to be coupled to a new syringe unit 2 for a further injection of the same or another medicament by means of the auto injector 1.

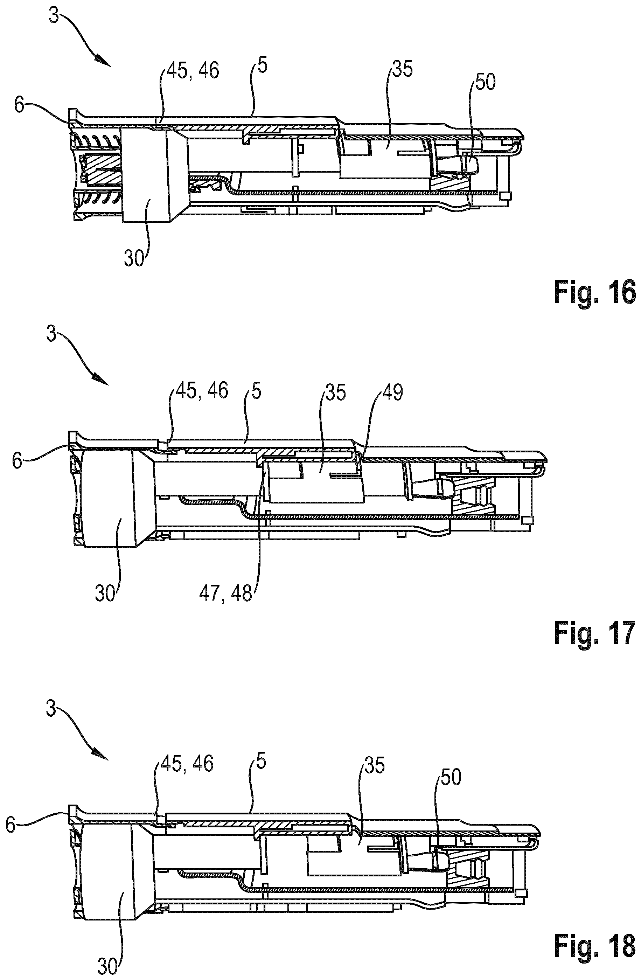

Figures 16 to 18 show length sections of the syringe unit 3 in different situations where the movement of the second sleeve 30 is blocked. Fig. 16 shows the syringe unit 3 in an unused state, i.e. prior to the connection of the syringe unit 3 to the drive unit 2. The second sleeve 30 is prevented from moving along the second central axis B in the second housing 5 by the engagement of the locking elements 50 with the inner wall of the second housing 5. Removal of the cap 6 is prevented as the disengagement of the third connection means 45 and of the fourth connection means 46 is blocked by the second sleeve 30.

Fig. 17 shows the syringe unit 3 after completion of the injection. The second sleeve 30 is connected with the follower element 35 by means of the fifth connection means 47 and the sixth connection means 48. The follower element 35 itself has been pulled along with the second sleeve 30 in the proximal direction, so that the hook elements 49 have engaged into matching recesses on the inner wall of the second housing 5. Hence, the second sleeve 30 is permanently blocked within the second housing 5 and may no longer be moved about the second length axis B. As the third connection means 45 and the fourth connection means 46 are not blocked by the second sleeve 30, the cap 6 may be re-connected to the proximal end of the second housing 5.

Fig. 18 shows the syringe unit 3 after an abortion of the injection. If the injection is aborted, the electronic control module 20 retracts the actuator 11 back into the initial position which leads to a distal movement of the first sleeve 10. Thereby, the proximal end of the first sleeve 10 disconnects from the distal end of the second sleeve 30. This disconnection leads to an engagement of the locking elements 50 with the inner wall of the second housing 5 thereby preventing any further movement of the second sleeve 30 within the second housing 5. Please note that the follower element 35 is not connected to the second sleeve 30 in this case.

### List of reference symbols

- 1: Auto injector
- 2: Drive unit
- 3: Syringe unit
- 4: First housing
- 5: Second housing
- 6: Cap
- 7: Button
- 8: Screen
- 9: Plug
- 10: First sleeve
- 11: Actuator
- 12: Stop surface
- 13: Threaded rod
- 14: Electric motor
- 15: Gear
- 16: First elastic element
- 17: Spring lock washer
- 18: Holder
- 19.1, 19.2: Secondary batteries
- 20: Electronic control module
- 21: Wireless communication module
- 22: First detector
- 23: Second detector
- 24: Third detector
- 25: Protective glass
- 26: Frame
- 27: Thermal element
- 28: Proximal
- 30: Second sleeve
- 31: Syringe
- 32: Needle
- 33: Barrel
- 34: Plunger
- 35; 35.1, 35.2: Follower element
- 36.1, 36.2: Abutment element
- 37: Rigid needle seal
- 38: Second elastic element
- 39: Distal section
- 40: Recess
- 41: Opening
- 42: Volume
- 43: First connection means
- 44: Second connection means
- 45: Third connection means
- 46: Fourth connection means
- 47: Fifth connection means
- 48: Sixth connection means
- 49: Hook element
- 50: Locking element
- 60: Skin

## Claims

1. An auto injector (1) for injection of a medicament, said auto injector (1) including a drive unit (2) as well as a syringe unit (3) which is releasably coupled to the drive unit (2), the drive unit (2) comprising:
a) a longitudinal first housing (4) with a first central axis and with first connection means (43) at a proximal end;
b) an actuator (11) being movable along the first central axis by means of a linear drive of the drive; and
c) a first sleeve (10) which is linearly movable within the first housing (4) along the first central axis, the sleeve (10) being biased towards the proximal end of the first housing (4) by means of a first elastic element (16), said first sleeve (10) being in contact with a stop surface (12) of the actuator (11) in an initial position thereof, said stop surface (12) limiting the movement of the first sleeve (10) towards the proximal end;
wherein the syringe unit (3) comprises:
d) a longitudinal second housing (5) with a second central axis and with second connection means (44) at a distal end thereof, said second connection means (44) being configure to releasably engage the first connection means (43) of the first housing (4), the second housing (5) further including third connection means (45) arranged at a proximal end thereof;
e) a syringe (31) arranged within said second housing (5);
f) a cap (6) which is releasably coupled to the proximal end of the second housing (5) by means of fourth connection means (46), said fourth connection means (46) being releasably engaged to the third connection means (45);
g) a second sleeve (30) which is linearly movable along the second central axis, said sleeve (30) blocking the release of the engagement of the third connection means (45) with the fourth connection means (46)
wherein the first sleeve (10) is adapted to block the release of the engagement of the first connection means (43) with the second connection means (44) when the actuator (11) is moved proximally from the initial position to a first position and to abut onto and to push the second sleeve (30) towards the proximal end of the second housing (5) such as to allow a release of the blocking of the coupling of the third connection means (45) with the forth connection means (46) when the actuator (11) is moved proximally from the first position to a second position.

2. The auto injector according to claim 1, **characterized in that** a proximal end of the second sleeve (30) is configured to move into the cap (6) to a first active position when the second sleeve (30) is pushed towards the proximal end of the second housing (5) by the first sleeve (10).

3. The auto injector according to claim 2, **characterized in that** the cap (6) comprises a second elastic element (38) which is compressed or is arranged to be compressed when the proximal end of the second sleeve (30) moves into the cap (6) towards the first active position.

4. The auto injector according to claim 3, **characterized in that** the first sleeve (10), the second sleeve (30) and the actuator (11) are dimensioned such that the first sleeve (10) is spaced apart from the stop surface (12) when the proximal end of the second sleeve (30) is in the first active position, and configure to be pushed into abutment with the stop surface (12) by means of the first elastic element (16) when the cap (6) is removed, allowing the proximal end of the second sleeve (30) to be pushed into a second active position.

5. The auto injector according to any of claims 1 to 4, **characterized in that** the second sleeve (30) comprises locking elements (50) which prevent movement of the second sleeve (30) when the distal end of the second sleeve (30) is spaced apart from the proximal end of the first sleeve (10) and which allow movement of the second sleeve (30) when the proximal end of the first sleeve (10) abuts on the distal end of the second sleeve (30).

6. The auto injector according to claim 4 or 5, **characterized in that** the second sleeve (30) comprises fifth connection means (47) at a distal end which are configured to engage with sixth connection (48) means of a follower element (35) which is arranged linearly movable along the second central axis in the second housing (5) when said second sleeve (30) is moved to a retracted position against the bias of the first elastic element (16) when the auto injector (1) is pressed onto the skin of a patient, thereby unsheathing the needle (32) for pricking the skin.

7. The auto injector according to claim 6, **characterized in that** said follower element (35) comprises hook elements (49) which are configured to engage into a permanent form-fit connection with retainer elements arranged on an inner wall of the second housing (5) when the second sleeve (30) is moved from the retracted position towards the second active position by means of the first elastic element (16) when the auto injector (1) is removed from the skin in order to permanently sheath the needle (32).

8. The auto injector according to any of claims 1 to 7, **characterized in that** the actuator (11) is configured to abut on a plunger (34) of the syringe (31) in the second position.

9. The auto injector according to any of claims 1 to 8, **characterized in that** the drive unit (2) comprises an electronic control module (20) as well as at least one detector (22) which detects when the first sleeve (10) reaches at least one defined position along the first central axis and/or whether the first connection means (43) are engaged with the second connection means (44) the at least one detector (22) preferably being a dome tact switch or a switch actuator.

10. The auto injector according to claims 9 **characterized in that** the electronic control module (20) comprises means to identify the syringe unit (3) coupled to the drive unit (2), said means preferably including an RFID reader.

11. The auto injector according to any of claims 9 or 10, **characterized in that** the drive unit (2) comprises output means to signal a status of the auto injector (1) to a patient, the output means preferably being a screen, at least one light indicator, a vibration generator and/or an audio signal generator.

## Patentansprüche

1. Autoinjektor (1) zum Injizieren eines Medikaments, wobei der Autoinjektor (1) eine Antriebseinheit (2) sowie eine Spritzeneinheit (3) einschließt, die lösbar mit der Antriebseinheit (2) gekoppelt ist, wobei die Antriebseinheit (2) umfasst:
a) ein längliches erstes Gehäuse (4) mit einer ersten Mittelachse und mit ersten Verbindungsmitteln (43) an einem proximalen Ende;
b) einen Aktuator (11), der mittels eines Linearantriebs des Antriebs entlang der ersten Mittelachse beweglich ist; und
c) eine erste Hülse (10), die innerhalb des ersten Gehäuses (4) entlang der ersten Mittelachse linear beweglich ist, wobei die Hülse (10) mittels eines ersten elastischen Elements (16) in Richtung des proximalen Endes des ersten Gehäuses (4) vorgespannt ist, wobei die erste Hülse (10) mit einer Anschlagoberfläche (12) des Aktuators (11) in einer Ausgangsposition davon in Kontakt ist, wobei die Anschlagoberfläche (12) die Bewegung der ersten Hülse (10) in Richtung des proximalen Endes begrenzt;
wobei die Spritzeneinheit (3) umfasst:
d) ein längliches zweites Gehäuse (5) mit einer zweiten Mittelachse und mit zweiten Verbindungsmitteln (44) an einem distalen Ende davon, wobei die zweiten Verbindungsmittel (44) konfiguriert sind, um lösbar mit den ersten Verbindungsmitteln (43) des ersten Gehäuses (4) in Eingriff zu kommen, wobei das zweite Gehäuse (5) ferner dritte Verbindungsmittel (45) einschließt, die an einem proximalen Ende davon angeordnet sind;
e) eine Spritze (31), die innerhalb des zweiten Gehäuses (5) angeordnet ist;
f) eine Kappe (6), die mittels vierter Verbindungsmittel (46) lösbar mit dem proximalen Ende des zweiten Gehäuses (5) gekoppelt ist, wobei die vierten Verbindungsmittel (46) lösbar mit den dritten Verbindungsmitteln (45) in Eingriff stehen;
g) eine zweite Hülse (30), die entlang der zweiten Mittelachse linear beweglich ist, wobei die Hülse (30) das Lösen des Eingriffs der dritten Verbindungsmittel (45) mit den vierten Verbindungsmitteln (46) blockiert;
wobei die erste Hülse (10) dazu ausgelegt ist, das Lösen des Eingriffs der ersten Verbindungsmittel (43) mit den zweiten Verbindungsmitteln (44) zu blockieren, wenn der Aktuator (11) proximal von der Ausgangsposition in eine erste Position bewegt wird, und so an der zweiten Hülse (30) anliegt und diese in Richtung des proximalen Endes des zweiten Gehäuses (5) drückt, dass ein Lösen der Blockierung der Kopplung der dritten Verbindungsmittel (45) mit den vierten Verbindungsmitteln (46) ermöglicht wird, wenn der Aktuator (11) proximal von der ersten Position in eine zweite Position bewegt wird.

2. Autoinjektor nach Anspruch 1, **dadurch gekennzeichnet, dass** ein proximales Ende der zweiten Hülse (30) konfiguriert ist, um sich in die Kappe (6) in eine erste aktive Position zu bewegen, wenn die zweite Hülse (30) durch die erste Hülse (10) in Richtung des proximalen Endes des zweiten Gehäuses (5) gedrückt wird.

3. Autoinjektor nach Anspruch 2, **dadurch gekennzeichnet, dass** die Kappe (6) ein zweites elastisches Element (38) umfasst, das komprimiert wird oder so angeordnet ist, dass es komprimiert wird, wenn sich das proximale Ende der zweiten Hülse (30) in die Kappe (6) in Richtung der ersten aktiven Position bewegt.

4. Autoinjektor nach Anspruch 3, **dadurch gekennzeichnet, dass** die erste Hülse (10), die zweite Hülse (30) und der Aktuator (11) so dimensioniert sind, dass die erste Hülse (10) von der Anschlagoberfläche (12) beabstandet ist, wenn sich das proximale Ende der zweiten Hülse (30) in der ersten aktiven Position befindet, und konfiguriert ist, um mittels des ersten elastischen Elements (16) in Anlage mit der Anschlagoberfläche (12) gedrückt zu werden, wenn die Kappe (6) entfernt wird, wodurch ermöglicht wird, dass das proximale Ende der zweiten Hülse (30) in eine zweite aktive Position gedrückt wird.

5. Autoinjektor nach einem der Ansprüche 1 bis 4,
**dadurch gekennzeichnet, dass** die zweite Hülse (30) Verriegelungselemente (50) umfasst, die eine Bewegung der zweiten Hülse (30) verhindern, wenn das distale Ende der zweiten Hülse (30) von dem proximalen Ende der ersten Hülse (10) beabstandet ist, und die eine Bewegung der zweiten Hülse (30) ermöglichen, wenn das proximale Ende der ersten Hülse (10) am distalen Ende der zweiten Hülse (30) anliegt.

6. Autoinjektor nach Anspruch 4 oder 5, **dadurch gekennzeichnet, dass** die zweite Hülse (30) an einem distalen Ende fünfte Verbindungsmittel (47) umfasst, die konfiguriert sind, um mit sechsten Verbindungsmitteln (48) eines Folgeelements (35) in Eingriff zu kommen, das linear beweglich entlang der zweiten Mittelachse in dem zweiten Gehäuse (5) angeordnet ist, wenn die zweite Hülse (30) gegen die Vorspannung des ersten elastischen Elements (16) in eine zurückgezogene Position bewegt wird, wenn der Autoinjektor (1) auf die Haut eines Patienten gedrückt wird, wodurch die Nadel (32) zum Einstechen der Haut enthüllt wird.

7. Autoinjektor nach Anspruch 6, **dadurch gekennzeichnet, dass** das Folgeelement (35) Hakenelemente (49) umfasst, die konfiguriert sind, um in eine dauerhafte formschlüssige Verbindung mit an einer Innenwand des zweiten Gehäuses (5) angeordneten Halteelementen einzugreifen, wenn die zweite Hülse (30) beim Entfernen des Autoinjektors (1) von der Haut mittels des ersten elastischen Elements (16) aus der zurückgezogenen Position in Richtung der zweiten aktiven Position bewegt wird, um die Nadel (32) dauerhaft zu umhüllen.

8. Autoinjektor nach einem der Ansprüche 1 bis 7,
**dadurch gekennzeichnet, dass** der Aktuator (11) konfiguriert ist, um in der zweiten Position an einem Kolben (34) der Spritze (31) anzuliegen.

9. Autoinjektor nach einem der Ansprüche 1 bis 8,
**dadurch gekennzeichnet, dass** die Antriebseinheit (2) ein elektronisches Steuermodul (20) sowie mindestens einen Detektor (22) umfasst, der erkennt, wann die erste Hülse (10) mindestens eine definierte Position entlang der ersten Mittelachse erreicht und/oder ob die ersten Verbindungsmittel (43) mit den zweiten Verbindungsmitteln (44) in Eingriff stehen, wobei der mindestens eine Detektor (22) vorzugsweise ein Kuppeltaktschalter oder ein Schaltaktor ist.

10. Autoinjektor nach Anspruch 9, **dadurch gekennzeichnet, dass** das elektronische Steuermodul (20) Mittel zum Identifizieren der mit der Antriebseinheit (2) gekoppelten Spritzeneinheit (3) umfasst, wobei die Mittel vorzugsweise einen RFID-Leser einschließen.

11. Autoinjektor nach einem der Ansprüche 9 oder 10,
**dadurch gekennzeichnet, dass** die Antriebseinheit (2) Ausgabemittel umfasst, um einem Patienten einen Status des Autoinjektors (1) zu signalisieren, wobei die Ausgabemittel vorzugsweise ein Bildschirm, mindestens eine Lichtanzeige, ein Vibrationsgenerator und/oder ein Audiosignalgenerator sind.

## Revendications

1. Auto-injecteur (1) pour l'injection d'un médicament, ledit auto-injecteur (1) comportant une unité d'entraînement (2) ainsi qu'une unité de seringue (3) qui est accouplée de manière amovible à l'unité d'entraînement (2), l'unité d'entraînement (2) comprenant :
a) un premier boîtier longitudinal (4) avec un premier axe central et avec un premier moyen de liaison (43) au niveau d'une extrémité proximale ;
b) un actionneur (11) étant mobile le long du premier axe central au moyen d'un entraînement linéaire de l'entraînement ; et
c) un premier manchon (10) qui est mobile de manière linéaire à l'intérieur du premier boîtier (4) le long du premier axe central, le manchon (10) étant sollicité vers l'extrémité proximale du premier boîtier (4) au moyen d'un premier élément élastique (16), ledit premier manchon (10) étant en contact avec une surface de butée (12) de l'actionneur (11) dans une position initiale de celui-ci, ladite surface de butée (12) limitant le mouvement du premier manchon (10) en direction de l'extrémité proximale ;
dans lequel l'unité de seringue (3) comprend :
d) un second boîtier longitudinal (5) avec un second axe central et avec un deuxième moyen de liaison (44) au niveau l'extrémité distale de celui-ci, ledit deuxième moyen de liaison (44) étant conçu pour venir en prise de manière amovible avec le premier moyen de liaison (43) du premier boîtier (4), le second boîtier (5) comportant en outre un troisième moyen de liaison (45) agencé au niveau de l'extrémité proximale de celui-ci ;
e) une seringue (31) agencée à l'intérieur dudit second boîtier (5) ;
f) un capuchon (6) qui est accouplé de manière amovible à l'extrémité proximale du second boîtier (5) au moyen d'un quatrième moyen de liaison (46), ledit quatrième moyen de liaison (46) étant mis en prise de manière amovible avec le troisième moyen de liaison (45) ;
g) un second manchon (30) qui peut être déplacé de manière linéaire le long du second axe central, ledit manchon (30) bloquant la libération de la mise en prise du troisième moyen de liaison (45) avec le quatrième moyen de liaison (46) ;
dans lequel le premier manchon (10) est adapté à bloquer la libération de la mise en prise du premier moyen de liaison (43) avec le deuxième moyen de liaison (44) lorsque l'actionneur (11) est déplacé de manière proximale de la position initiale à une première position et pour venir en butée contre et pousser le second manchon (30) vers l'extrémité proximale du second boîtier (5) de manière à permettre une libération du blocage de l'accouplement du troisième moyen de liaison (45) avec le quatrième moyen de liaison (46) lorsque l'actionneur (11) est déplacé de manière proximale de la première position à une seconde position.

2. Auto-injecteur selon la revendication 1, **caractérisé en ce qu'**une extrémité proximale du second manchon (30) est conçue pour se déplacer dans le capuchon (6) jusqu'à une première position active lorsque le second manchon (30) est poussé vers l'extrémité proximale du second boîtier (5) par le premier manchon (10).

3. Auto-injecteur selon la revendication 2, **caractérisé en ce que** le capuchon (6) comprend un second élément élastique (38) qui est comprimé ou est agencé pour être comprimé lorsque l'extrémité proximale du second manchon (30) se déplace dans le capuchon (6) vers la première position active.

4. Auto-injecteur selon la revendication 3, **caractérisé en ce que** le premier manchon (10), le second manchon (30) et l'actionneur (11) sont dimensionnés de telle sorte que le premier manchon (10) est espacé de la surface de butée (12) lorsque l'extrémité proximale du second manchon (30) est dans la première position active, et conçu pour être poussé en butée contre la surface de butée (12) au moyen du premier élément élastique (16) lorsque le capuchon (6) est retiré, permettant à l'extrémité proximale du second manchon (30) d'être poussée dans une seconde position active.

5. Auto-injecteur selon l'une quelconque des revendications 1 à 4, **caractérisé en ce que** le second manchon (30) comprend des éléments de verrouillage (50) qui empêchent le mouvement du second manchon (30) lorsque l'extrémité distale du second manchon (30) est éloignée de l'extrémité proximale du premier manchon (10) et qui permettent le mouvement du second manchon (30) lorsque l'extrémité proximale du premier manchon (10) vient en butée contre l'extrémité distale du second manchon (30).

6. Auto-injecteur selon la revendication 4 ou 5, **caractérisé en ce que** le second manchon (30) comprend un cinquième moyen de liaison (47) au niveau d'une extrémité distale qui est conçu pour venir en prise avec un sixième moyen de liaison (48) d'un élément suiveur (35) qui est agencé de manière linéaire mobile le long du second axe central dans le second boîtier (5) lorsque ledit second manchon (30) est déplacé vers une position rétractée contre la sollicitation du premier élément élastique (16) lorsque l'auto-injecteur (1) est pressé sur la peau d'un patient, dégainant ainsi l'aiguille (32) pour piquer la peau.

7. Auto-injecteur selon la revendication 6, **caractérisé en ce que** ledit élément suiveur (35) comprend des éléments crochets (49) qui sont conçus pour venir en prise avec une liaison par complémentarité de formes permanente avec des éléments de retenue agencés sur une paroi intérieure du second boîtier (5) lorsque le second manchon (30) est déplacé de la position rétractée vers la seconde position active au moyen du premier élément élastique (16) lorsque l'auto-injecteur (1) est retiré de la peau afin de gainer de façon permanente l'aiguille (32).

8. Auto-injecteur selon l'une quelconque des revendications 1 à 7, **caractérisé en ce que** l'actionneur (11) est conçu pour venir en butée contre un piston (34) de la seringue (31) dans la seconde position.

9. Auto-injecteur selon l'une quelconque des revendications 1 à 8, **caractérisé en ce que** l'unité d'entraînement (2) comprend un module de commande électronique (20) ainsi qu'au moins un détecteur (22) qui détecte lorsque le premier manchon (10) atteint au moins une position définie le long du premier axe central et/ou si le premier moyen de liaison (43) est mis en prise avec le deuxième moyen de liaison (44), l'au moins un détecteur (22) étant de préférence un interrupteur à contact en dôme ou un actionneur d'interrupteur.

10. Auto-injecteur selon la revendication 9, **caractérisé en ce que** le module de commande électronique (20) comprend un moyen d'identifier l'unité de seringue (3) couplée à l'unité d'entraînement (2), ledit moyen comportant de préférence un lecteur RFID.

11. Auto-injecteur selon l'une quelconque des revendications 9 ou 10, **caractérisé en ce que** l'unité d'entraînement (2) comprend un moyen de sortie pour signaler un état de l'auto-injecteur (1) à un patient, le moyen de sortie étant de préférence un écran, au moins un indicateur lumineux, un générateur de vibrations et/ou un générateur de signaux audio.
